# EUROPEAN PATENT APPLICATION

(11) **EP 4 361 276 A1**
(43) Date of publication of application: **01.05.2024**
(21) Application number: 22828704.1
(22) Date of filing: 21.06.2022
(51) Int. Cl.: C12N 15/70, C12N 9/02, C12N 9/10, C12P 13/06, C12P 13/12

(54) **RECOMBINANT MICROORGANISM IN WHICH EXPRESSION OF NADH:QUINONE OXIDOREDUCTASE IS CONTROLLED, AND METHOD FOR PRODUCING O-PHOSPHOSERINE, CYSTEINE, AND DERIVATIVE THEREOF BY USING SAME**

(30) Priority: 23.06.2021 KR 20210081785
(71) Applicant: CJ Cheiljedang Corporation, Seoul 04560 (KR)
(72) Inventor: JUNG, Hwi-Min, Seoul 04560 (KR); PARK, Hye Min, Seoul 04560 (KR); SIM, Hee-jin, Seoul 04560 (KR); LEE, Jin Nam, Seoul 04560 (KR)
(74) Representative: Vossius & Partner Patentanwälte Rechtsanwälte mbB
(86) International application number: PCT/KR2022/008745
(87) International publication number: WO 2022/270857

(57) **Abstract**

The present application relates to a recombinant microorganism, in which the expression of NADH:quinone oxidoreductase is regulated, and a method for producing O-phosphoserine, cysteine, and cysteine derivatives using the same.

## Description

### [Technical Field]

The present application relates to a recombinant microorganism, in which the expression of NADH:quinone oxidoreductase is regulated, and a method for producing O-phosphoserine, cysteine, and cysteine derivatives using the same.

### [Background Art]

L-Cysteine is an amino acid that has an important role in sulfur metabolism in all living organisms. It is used in the synthesis of proteins, such as hair keratin, *etc.,* glutathione, biotin, methionine, and other sulfur-containing metabolites, and also serves as a precursor for coenzyme A biosynthesis. Known methods of producing L-cysteine using microorganisms include: 1) a method of biologically converting D,L-2-aminothiazoline-4-carboxylic acid to L-cysteine using microorganisms, 2) a method of producing L-cysteine by direct fermentation using E. *coli* (EP 0885962 B; Wada, M. and Takagi, H., Appl. Microbiol. Biochem., 73:48-54, 2006), and 3) a method of producing O-phosphoserine by fermentation using microorganisms, and converting O-phosphoserine into L-cysteine by reacting *O*-phosphoserine with a sulfide under the catalytic action of *O*-phosphoserine sulfhydrylase (US 2012-0190081 A1).

In particular, for the production of cysteine by way of method 3) in high yield, the precursor, *O*-phosphoserine, should be produced in an excess amount.

### [Disclosure]

### [Technical Problem]

The technical problem of the present application is to provide a recombinant microorganism, in which the expression of NADH:quinone oxidoreductase is regulated, and a method for producing O-phosphoserine, cysteine, and cysteine derivatives using the same.

### [Technical Solution]

One object of the present application is to provide a recombinant microorganism of the genus *Escherichia* having an enhanced NADH:quinone oxidoreductase activity and an *O*-phosphoserine producing ability.

Another object of the present application is to provide a method for producing *O*-phosphoserine, including: culturing the *O*-phosphoserine-producing microorganism of the present application in a medium.

Still another object of the present application is to provide a method for producing cysteine or a derivative thereof, including:
a) culturing an *O*-phosphoserine-producing microorganism with an enhanced NADH:quinone oxidoreductase activity in a medium to produce *O*-phosphoserine or a medium containing the same; and
b) reacting *O*-phosphoserine produced in step a) or a medium containing the same with a sulfide in the presence of *O*-phosphoserine sulfhydrylase (OPSS) or a microorganism expressing the same.

### [Advantageous Effects]

The OPS-producing microorganism having an enhanced NADH:quinone oxidoreductase activity of the present application can produce OPS with high efficiency.

### [Detailed Description of Preferred Embodiments]

The present application will be described in detail as follows. Meanwhile, each description and embodiment disclosed herein can be applied to each other description and embodiment. That is, all combinations of various elements disclosed herein fall within the scope of the present application. Further, the scope of the present application is not limited by the specific description described below. Additionally, a number of papers and patent documents have been cited throughout the present specification. The content of the cited papers and patent documents is incorporated herein by reference in their entirety and the level of technical field to which the present application belongs and the contents of the present application will be described more clearly.

One aspect of the present application provides an *O*-phosphoserine-producing microorganism having an enhanced NADH:quinone oxidoreductase activity.

As used herein, the term "*O*-phosphoserine" (hereinafter, "OPS") refers to a phosphoric acid ester of serine, which serves as a constituting component for many proteins. In particular, the OPS is a precursor of L-cysteine and can be converted to cysteine by reacting with a sulfide under the catalytic action of OPS sulfhydrylase (hereinafter, "OPSS"), but is not limited thereto (US 2012-0190081 A1).

As used herein, the term "NADH:quinone oxidoreductase" (hereinafter, "Nuo") refers to an enzyme that reduces quinone in the cell membrane by oxidizing NADH in the electron transport chain of microorganisms. The enzyme protein may also be named NADH dehydrogenase-1 (NDH-1). The gene encoding the protein may be, for example, the nuoABCEFGHIJKLMN gene cluster, but is not limited thereto. The nuoABCEFGHIJKLMN gene cluster constitutes the nuo operon, and the expression thereof can be regulated by a promoter in front of the operon and a polynucleotide in the ribosome binding site. In the present application, the "nuoABCEFGHIJKLMN gene" may be used interchangeably with "gene encoding NADH:quinone oxidoreductase", "nuoABCEFGHIJKLMN gene", "nuo operon", and "nuo gene".

In the present application, the "operon" refers to a functional unit of DNA including a group of genes whose expression is regulated by one expression regulatory sequence, specifically one promoter. The mRNA transcribed by the operon may be a polycistronic mRNA in which a single mRNA molecule encodes one or more proteins, or a monocistronic mRNA in which a single mRNA molecule encodes one protein.

The Nuo is a complex of 13 subunit proteins (NuoA, NuoB, NuoC, NuoE, NuoF, NuoG, NuoH, Nuol, NuoJ, NuoK, NuoL, NuoM, NuoN). The two types of operons, nuoABCEFGHIJKL and nuoMN, are used as template in the translation of each subunit protein. The structure of the nuo operon can be found in EcoCyc (www.biocyc.org) (Accession No. EG12082). The nuo operon is known to include a structural gene and an expression regulatory region. The "expression regulatory region" of the nuo operon refers to a region that exists upstream of the structural gene constituting the nuo operon and thus can regulate the expression of the structural gene. The expression regulatory region of the nuo operon may include a promoter (nuoA promoter and/or nuoM promoter) excluding the structural gene and an operator, and may specifically include a promoter.

The nuo operon may include a nucleotide sequence encoding an amino acid sequence having a homology or identity of 70%, 80%, 85%, 90%, 95%, 96%, 97%, 98%, or 99% or more to the amino acid sequences of SEQ ID NO: 26 to SEQ ID NO: 38. Specifically, the nuo operon may include a structural gene encoding the amino acid sequence of SEQ ID NOS: 26 to 38 or a homology or identity thereto and exhibiting the corresponding function; and an expression regulatory region that regulates the expression of the sequence of the structural gene. The sequences of SEQ ID NOS: 26 to 38 can be confirmed in NCBI Genbank, a known database.

Specifically, the nuo operon may be a nucleotide sequence having SEQ ID NO: 1 and/or having a homology or identity of 70%, 80%, 85%, 90%, 95%, 96%, 97%, 98%, or 99% or more to SEQ ID NO: 1. Additionally, it is apparent that any nucleotide sequence, in which part of the sequence is deleted, modified, substituted, or added, may also fall within the scope of the present application as long as the nucleotide sequence has such a homology or identity and exhibits a function corresponding to that of the nuo operon.

As used herein, the term "homology and identity" refer to a degree of relatedness between two given amino acid sequences or nucleotide sequences, and may be expressed as a percentage. The terms homology and identity may often be used interchangeably with each other.

The sequence homology or identity of conserved polynucleotide or polypeptides may be determined by standard alignment algorithms and can be used with a default gap penalty established by the program being used. Substantially, homologous or identical sequences are generally expected to hybridize to all or at least about 50%, 60%, 70%, 80%, or 90% of the entire length of the sequences under moderate or highly stringent conditions. Polynucleotides that contain degenerate codons instead of codons in hybridizing polynucleotides are also considered.

The homology or identity of the polypeptide or polynucleotide sequences may be determined by, for example, BLAST algorithm according to the literature (see Karlin and Altschul, Pro. Natl. Acad. Sci. USA, 90, 5873 (1993)), or FASTA by Pearson (see Methods Enzymol., 183, 63, 1990). Based on the algorithm BLAST, a program known as BLASTN or BLASTX has been developed (see: http://www.ncbi.nlm.nih.gov). Further, whether any amino acid or polynucleotide sequences have a homology, similarity, or identity with each other, it may be identified by comparing the sequences in a Southern hybridization experiment under stringent conditions as defined, and appropriate hybridization conditions defined are within the skill of the art, and may be determined by a method well known to those skilled in the art (*e.g.,* J. Sambrook et al., Molecular Cloning, A Laboratory Manual, 2nd Edition, Cold Spring Harbor Laboratory press, Cold Spring Harbor, New York, 1989; F. M. Ausubel et al., Current Protocols in Molecular Biology*).*

The microorganism of the present application is not limited by its type as long as it can produce OPS, and may be any prokaryotic or eukaryotic microorganism, specifically a prokaryotic microorganism. The prokaryotic microorganism may include microbial strains belonging to the genus *Escherichia,* the genus *Erwinia,* the genus *Serratia,* the genus *Providencia,* the genus *Corynebacterium,* and the genus *Brevibacterium,* specifically a microorganism belonging to the genus *Escherichia,* and more specifically *Escherichia coli,* but is not limited thereto. In particular, in the case of the microorganism belonging to the genus *Escherichia,* OPS and L-serine can be produced through SerA, SerC, and SerB, which are enzymes of the biosynthetic pathway of L-serine (Ahmed Zahoor, Computational and structural biotechnology journal, Vol. 3, 2012 October; Wendisch V. F. et al., Curr Opin Microbiol. 2006 Jun;9(3):268-74; Peters-Wendisch P. et al., Appl Environ Microbiol. 2005 Nov;7 1(II):7 139-44.).

The "*O*-phosphoserine-producing microorganism" of the present application refers to a microorganism having the ability to naturally produce *O*-phosphoserine or a microorganism in which the *O*-phosphoserine producing ability is imparted to a parent strain that does not have the ability to produce *O*-phosphoserine. Specifically, the microorganism may be an *O*-phosphoserine-producing microorganism with an enhanced Nuo activity due to natural or artificial genetic modification. For the purpose of the present application, the *O*-phosphoserine-producing microorganism may be any microorganism capable of producing *O*-phosphoserine by enhancing Nuo activity by the method disclosed in the present application. In the present application, the "*O*-phosphoserine-producing microorganism" may be used interchangeably with "microorganism producing *O*-phosphoserine" or "microorganism having *O-*phosphoserine producing ability".

In one embodiment, the *O*-phosphoserine-producing microorganism of the present application may be a genetically modified microorganism or a recombinant microorganism in which the activity of Nuo is enhanced, thereby increasing the desired *O*-phosphoserine producing ability, but is not limited thereto.

As used herein, the term "enhancement of activity" of a protein means that the activity of a protein is increased compared to its endogenous activity. The "endogenous activity" refers to the activity of a particular protein originally possessed by a parent strain before transformation or a non-modified microorganism, when a trait is altered through genetic modification due to natural or artificial factors, and may be used interchangeably with "activity before modification". The "increase" in the activity of a protein compared to its endogenous activity means that the activity of the protein is enhanced compared to that of a particular protein originally possessed by a parent strain before transformation or a non-modified microorganism. For example, the parent strain may be *Escherichia coli* ATCC27325. As another example, the parent strain may be a strain including a modification to increase OPS producing ability, for example, CA07-0012 (KCCM 11212P; US 2012-0190081 A) or CA07-4821, but is not limited thereto.

The "enhancement of activity" may be achieved by introducing a foreign protein, or by enhancing the activity of the endogenous protein, and may specifically be achieved by enhancing the activity of the endogenous protein. The enhancement of the activity of the protein can be confirmed by the increase in the level of activity of the protein, expression level, or the amount of product produced from the protein.

The enhancement of the activity can be applied by way of various methods well known in the art, and is not limited as long as it can enhance the activity of the target protein compared to that of the microorganism before modification. Specifically, genetic engineering and/or protein engineering well known to those skilled in the art, which is a common method of molecular biology, may be used, but the method is not limited thereto (*e.g.,* Sitnicka et al. Functional Analysis of Genes. Advances in Cell Biology. 2010, Vol. 2. 1-16, Sambrook et al. Molecular Cloning 2012, *etc*.).

In the present application, the protein targeted for the activity enhancement, that is, the target protein, may be Nuo, but the protein is not limited as long as it is "a protein that forms a proton motive force by consuming NADH" or "a protein consuming NADH" and both proteins are encoded by the nuo operon.

The enhancement of Nuo activity of the present application may include enhancement of the activity of one or more of the subunit proteins constituting the Nuo protein complex.

Specifically, the enhancement of the activity of the protein of the present application may be achieved by:
1) increasing the intracellular copy number of a gene encoding the protein;
2) modifying the expression regulatory region of a gene encoding the protein on the chromosome;
3) modifying the nucleotide sequence encoding the initiation codon or 5'-UTR of the gene transcript encoding the protein;
4) modifying the amino acid sequence such that the activity of the protein is enhanced;
5) modifying the polynucleotide sequence encoding the protein such that the activity of the protein is enhanced (*e.g.*, modifying the gene sequence encoding the protein to encode a protein that has been modified to enhance the activity);
6) introducing a foreign polynucleotide exhibiting the activity of the protein or a codon-optimized variant polynucleotide of the polynucleotide;
7) codon-optimization of the polynucleotide encoding the protein;
8) analyzing the tertiary structure of the protein and thereby selecting and modifying the exposed site, or chemically modifying the same; or
9) a combination of two or more selected from above 1 to 8), but is not limited thereto.

Specifically, the 1) method of increasing the intracellular copy number of a gene encoding the protein may be achieved by way of any method known in the art, for example, by introducing a vector, which is operably linked to the gene encoding the protein and is able to replicate and function regardless of a host cell, into the host cell. Additionally, the method may be performed by introducing a vector, which is operably linked to the gene and is able to insert the gene into the chromosome of a host cell, into the host cell, but is not limited thereto.

As used herein, the term "vector" refers to a DNA construct containing the polynucleotide sequence encoding the target protein operably linked to a suitable regulatory sequence so as to be able to express the target protein in a suitable host cell. The expression regulatory sequence may include a promoter capable of initiating transcription, any operator sequence for regulating the transcription, a sequence encoding a suitable mRNA ribosome binding site, and a sequence for regulating termination of transcription and translation. Once transformed into a suitable host cell, the vector may replicate or function independently from the host genome, or may integrate into genome thereof.

The vector used in the present application is not particularly limited as long as it can be replicated, and any vector known in the art may be used. Examples of the vector typically used may include natural or recombinant plasmids, cosmids, viruses, and bacteriophages. For example, as a phage vector or cosmid vector, pWE15, M13, MBL3, MBL4, IXII, ASHII, APII, t10, t11, Charon4A, and Charon21A, *etc.* may be used; and as a plasmid vector, those based on pBR, pUC, pBluescriptll, pGEM, pTZ, pCL, pSK, pSKH and pET, *etc.* may be used. Specifically, pCL, pSK, pSKH130, pDZ, pACYC177, pACYC184, pECCG117, pUC19, pBR322, pMW118, pCC1BAC vectors, *etc.* may be used.

The insertion of the polynucleotide into the chromosome may be performed by way of any method known in the art, for example, homologous recombination, but is not limited thereto.

As used herein, the term "transformation" refers to the introduction of a recombinant vector containing a polynucleotide encoding a target protein into a host cell so that the protein encoded by the polynucleotide can be expressed in the host cell. As long as the transformed polynucleotide can be expressed in the host cell, it does not matter whether the transformed polynucleotide is integrated into the chromosome of the host cell and located therein or located extrachromosomally, and both cases can be included. The method for transforming the vector includes any method of introducing a nucleic acid into a cell, and may be performed by selecting a suitable standard technique as known in the art depending on the host cell. For example, the transformation may be carried out via electroporation, calcium phosphate (CaPO₄) precipitation, calcium chloride (CaCl₂) precipitation, microinjection, a polyethylene glycol (PEG) technique, a DEAE-dextran technique, a cationic liposome technique, a lithium acetate-DMSO technique, *etc.,* but the method is not limited thereto.

Further, as used herein, the term "operably linked" means that the polynucleotide sequence is functionally linked to a promoter sequence or expression regulatory region that initiates and mediates transcription of the polynucleotide encoding the target protein of the present application. The operable linkage may be prepared using a genetic recombinant technology well known in the art, and site-specific DNA cleavage and linkage may be prepared using cleavage and linking enzymes, *etc.* known in the art, but is not limited thereto.

The 2) method of modifying the expression regulatory sequence of a gene encoding the protein on the chromosome may be achieved by way of any method known in the art, for example, by inducing a modification on the sequence through deletion, insertion, non-conservative or conservative substitution of the nucleic acid sequence, or a combination thereof to further enhance the activity of the expression regulatory sequence or by replacing the sequence with a nucleic acid sequence having a stronger activity or inserting the same. The expression regulatory sequence may include, but is not particularly limited to, a promoter, an operator sequence, a sequence encoding a ribosome binding site, and a sequence regulating the termination of transcription and translation, *etc.* The method may specifically include inserting a strong heterologous promoter downstream of the original promoter, but is not limited thereto.

Examples of the known strong promoter may include CJ1 to CJ7 promoters (US 7662943 B2), lac promoter, trp promoter, trc promoter, tac promoter, lambda phage PR promoter, PL promoter, tet promoter, and rmf promoter, *etc.,* but are not limited thereto, and may include all substitutions with a stronger promoter compared to its endogenous activity.

The 3) method of modifying the nucleotide sequence of the initiation codon or 5'-UTR of the gene transcript encoding the protein may be achieved by way of any method known in the art, for example, by substituting the endogenous initiation codon of the protein with another initiation codon having a higher expression rate of the protein compared to the endogenous initiation codon, but is not limited thereto.

The 4) and 5) methods of modifying the amino acid sequence or the polynucleotide sequence may be achieved by way of any method known in the art, for example, by inducing a modification on the sequence through deletion, insertion, non-conservative or conservative substitution of the polynucleotide sequence, or a combination thereof to further enhance the activity of the polynucleotide sequence, or by replacing the sequence with a polynucleotide sequence modified to have a stronger activity. The replacement may specifically be performed by inserting the gene into the chromosome via homologous recombination, but is not limited thereto.

The vector used herein may further include a selection marker to confirm the insertion into the chromosome. The selection marker is for selecting the cells transformed with the vector, that is, for confirming the insertion of the gene to be introduced, and markers that provide selectable phenotypes, such as drug resistance, auxotrophy, resistance to cell toxic agents, or expression of surface proteins, may be used, but the selection marker is not limited thereto. Only cells expressing the selection marker are able to survive or to show different phenotypes in the environment treated with the selective agent, and thus the transformed cells may be selected.

The 6) method of introducing a foreign polynucleotide having the activity of the protein may be achieved by way of any method known in the art, for example, by introducing into a host cell a foreign polynucleotide encoding a protein that exhibits the same or similar activity to the protein or a codon-optimized variant polynucleotide thereof. The foreign polynucleotide may be used without limitation regardless of its origin or sequence as long as it exhibits the same or similar activity to the protein. In addition, the introduced foreign polynucleotide may be introduced into the host cell by optimizing its codons so that the optimized transcription and translation are achieved in the host cell. The introduction may be performed by those of ordinary skill in the art by appropriately selecting a transformation method known in the art, and the expression of the introduced polynucleotide in the host cell enables to produce the protein, thereby increasing its activity.

The 7) method of codon-optimization of the polynucleotide encoding the protein may be achieved by codon-optimization of an endogenous polynucleotide to increase the transcription or translation within a host cell, or by optimizing its codons such that the optimized transcription and translation of the foreign polynucleotide can be achieved within the host cell.

The 8) method of analyzing the tertiary structure of the protein and thereby selecting and modifying the exposed site, or chemically modifying the same may be achieved, for example, by comparing the sequence information of the polypeptide to be analyzed with a database, in which the sequence information of known proteins is stored, to determine template protein candidates according to the degree of sequence similarity, and thus confirming the structure based on the information to thereby select and transform or modify the exposed site to be modified or chemically modified.

In one embodiment, the enhancement of the activity of the protein may be achieved by way of any one or more of the above 1) to 2).

In any one of the above-described embodiments, the enhancement of the NADH:quinone oxidoreductase activity of the present application may be an increase in the expression of the nuo operon. In any one of the above-described embodiments, the enhancement of the NADH:quinone oxidoreductase activity may include a gene expression regulatory sequence with enhanced activity in the upstream of the gene encoding the same. Specifically, the upstream of the gene encoding the NADH:quinone oxidoreductase may be upstream of nuoA gene. In one embodiment, the enhancement of the NADH:quinone oxidoreductase activity may be an enhancement of the expression of one or more structural genes present in the operon, for example 2 or more, 3 or more, or the entire structural gene sequence by modifying one expression regulatory sequence present in the nuo operon encoding the Nuo protein complex. Specifically, the modification of the expression regulatory sequence may be an additional insertion of a gene expression regulatory sequence with enhanced activity between the endogenous promoter of the nuo operon and the nuoA gene; for example, the gene expression regulatory sequence may be a promoter, but is not limited thereto.

Such enhancement of the protein activity may mean that the activity or concentration of the protein is increased relative to the activity or concentration of the protein expressed in a wild-type or a microorganism before modification, or that the amount of product produced from the protein is increased, but is not limited thereto.

As used herein, the term "strain before modification" or "microorganism before modification" does not exclude a strain containing a mutation that may occur naturally in a microorganism, and may refer to a natural-type strain itself, or a strain before the trait is altered due to genetic modification caused by natural or artificial factors. In the present application, the modification of the traits may be an enhancement of the Nuo activity. The "strain before modification" or "microorganism before modification" may be used interchangeably with "non-mutant strain", "non-modified strain", "non-mutant microorganism", "non-modified microorganism", or "reference microorganism".

The microorganism of the present application further enhances the ability to produce OPS and/or the ability to excrete the OPS out of the cell; or may include modifications that enhance OPS decomposing ability and/or influx ability.

Examples of the modifications that enhance the ability to produce OPS and/or the ability to export OPS out of the cells, or enhance the OPS-decomposing and/or influx ability, may include attenuation of activity of phosphoserine phosphotase (SerB), enhancement of activity of phosphoserine excretion protein (YhhS), or a combination of these modifications, but are not limited thereto.

As used herein, the term "weakening" of a polypeptide is a comprehensive concept including both reduced or no activity compared to its endogenous activity. The weakening may be used interchangeably with terms such as inactivation, deficiency, down-regulation, decrease, reduce, attenuation, *etc.*

The weakening may also include a case where the polypeptide activity itself is decreased or removed compared to the activity of the polypeptide originally possessed by a microorganism due to a mutation of the polynucleotide encoding the polypeptide; a case where the overall level of intracellular polypeptide activity and/or concentration (expression level) is decreased compared to a natural strain due to the inhibition of expression of the gene of the polynucleotide encoding the polypeptide, or the inhibition of translation into the polypeptide, *etc*.; a case where the polynucleotide is not expressed at all; and/or a case where no polypeptide activity is observed even when the polynucleotide is expressed. As used herein, the term "endogenous activity" refers to the activity of a particular polypeptide originally possessed by a parent strain before transformation, a wild-type or a non-modified microorganism, when a trait is altered through genetic modification caused by natural or artificial factors, and may be used interchangeably with "activity before modification". The expression that the polypeptide activity is "inactivated, deficient, decreased, down-regulated, reduced or attenuated" compared to its endogenous activity means that the polypeptide activity is decreased compared to the activity of a particular polypeptide originally possessed by a parent strain before transformation or a non-modified microorganism.

The weakening of the polypeptide activity can be performed by way of any method known in the art, but the method is not limited thereto, and can be achieved by applying various methods well known in the art (*e.g.,* Nakashima N et al., Bacterial cellular engineering by genome editing and gene silencing. Int J Mol Sci. 2014;15(2):2773-2793, Sambrook et al. Molecular Cloning 2012, *etc*.)*.*

Specifically, the weakening of the polypeptide activity of the present application may be achieved by:
1) deleting a part or all of the gene encoding the polypeptide;
2) modifying the expression regulatory region (expression regulatory sequence) such that the expression of the gene encoding the polypeptide is decreased;
3) modifying the amino acid sequence constituting the polypeptide such that the polypeptide activity is removed or weakened (*e.g*., deletion/substitution/addition of one or more amino acids on the amino acid sequence);
4) modifying the gene sequence encoding the polypeptide such that the polypeptide activity is removed or weakened (*e.g.*, deletion/substitution/addition of one or more of nucleotides on the nucleotide sequence of the polypeptide gene to encode a polypeptide that has been modified to remove or weaken the activity of the polypeptide);
5) modifying the nucleotide sequence encoding the initiation codon or 5'-UTR of the gene transcript encoding the polypeptide;
6) introducing an antisense oligonucleotide (*e.g.*, antisense RNA), which binds complementary to the gene transcript encoding the polypeptide;
7) adding a sequence complementary to the Shine-Dalgarno (SD) sequence on the front end of the SD sequence of the gene encoding the polypeptide to form a secondary structure, thereby inhibiting the ribosomal attachment;
8) a reverse transcription engineering (RTE), which adds a promoter, which is to be reversely transcribed, on the 3' terminus of the open reading frame (ORF) of the gene sequence encoding the polypeptide; or
9) a combination of two or more selected from the methods 1) to 8) above, but is not particularly limited thereto.

For example,
The 1) method of deleting a part or all of the gene encoding the polypeptide may be achieved by deleting all of the polynucleotide encoding the endogenous target polypeptide within the chromosome, or by replacing the polynucleotide with a polynucleotide having a partially deleted nucleotide, or with a marker gene.

The 2) method of modifying the expression regulatory region (expression regulatory sequence) may be achieved by inducing a modification on the expression regulatory region (expression regulatory sequence) through deletion, insertion, non-conservative substitution or conservative substitution, or a combination thereof; or by replacing the sequence with a sequence having a weaker activity. The expression regulatory region may include a promoter, an operator sequence, a sequence encoding a ribosome binding site, and a sequence for regulating the termination of transcription and translation, but is not limited thereto.

Further, the 5) method of modifying the nucleotide sequence encoding the initiation codon or 5'-UTR of the gene transcript encoding the polypeptide may be achieved, for example, by substituting the nucleotide sequence with a nucleotide sequence encoding another initiation codon having a lower polypeptide expression rate than the endogenous initiation codon, but is not limited thereto.

The 3) and 4) methods of modifying the amino acid sequence or the polynucleotide sequence may be achieved by inducing a modification on the sequence through deletion, insertion, non-conservative or conservative substitution of the amino acid sequence of the polypeptide or the polynucleotide sequence encoding the polypeptide, or a combination thereof to weaken the activity of the polypeptide, or by replacing the sequence with an amino acid sequence or a polynucleotide sequence modified to have a weaker activity, or an amino acid sequence or a polynucleotide sequence modified to have no activity, but are not limited thereto. For example, the expression of the gene may be inhibited or weakened by introducing a mutation into the polynucleotide sequence to form a termination codon, but is not limited thereto.

The 6) method of introducing an antisense oligonucleotide (e.g., antisense RNA), which binds complementary to the gene transcript encoding the polypeptide can be found in the literature [Weintraub, H. et al., Antisense-RNA as a molecular tool for genetic analysis, Reviews - Trends in Genetics, Vol. 1(1) 1986].

The 7) method of adding a sequence complementary to the Shine-Dalgarno (SD) sequence on the front end of the SD sequence of the gene encoding the polypeptide to form a secondary structure, thereby inhibiting the ribosome attachment may be achieved by inhibiting mRNA translation or reducing the speed thereof.

The 8) reverse transcription engineering (RTE), which adds a promoter, which is to be reversely transcribed, on the 3' terminus of the open reading frame (ORF) of the gene sequence encoding the polypeptide may be achieved by forming an antisense nucleotide complementary to the gene transcript encoding the polypeptide to weaken the activity.

The SerB of the present application has an activity of converting OPS to L-serine, and thus the microorganism modified to weaken the SerB activity has the property of accumulating OPS therein, thus being useful for the production of OPS. The SerB of the present application may be a protein having or including an amino acid sequence represented by SEQ ID NO: 2, or may be a protein consisting or consisting essentially of an amino acid sequence represented by SEQ ID NO: 2, but is not limited thereto. Additionally, the SerB of the present application may have or include an amino acid sequence having a sequence homology or identity of 70%, 80%, 90%, 95%, or 99% or higher to the amino acid sequence represented by SEQ ID NO: 2, as long as it shows the SerB activity. Moreover, the SerB of the present application may consist or consist essentially of an amino acid sequence having a homology or identity of 70%, 80%, 90%, 95%, or 99% or higher to the amino acid sequence represented by SEQ ID NO: 2, but is not limited thereto. In addition, the polynucleotide encoding the SerB may have or include a nucleotide sequence of SEQ ID NO: 3 encoding the amino acid sequence represented by SEQ ID NO: 2. Further, the polynucleotide encoding the SerB may consist or consist essentially of a nucleotide sequence encoding the amino acid sequence represented by SEQ ID NO: 2. The polynucleotide encoding SerB of the present application may undergo various modifications in the coding region within the scope that does not change the amino acid sequence of the SerB protein, due to codon degeneracy or in consideration of the codons preferred in an organism in which the SerB protein is to be expressed. The polynucleotide encoding SerB of the present application may have or include a nucleotide sequence having a homology or identity of 70%, 80%, 90%, 95%, or 99% or higher, and less than 100% to the nucleotide sequence of SEQ ID NO: 3. Additionally, the polynucleotide encoding SerB of the present application may consist or consist essentially of a nucleotide sequence having a homology or identity of 70%, 80%, 90%, 95%, or 99% or higher, and less than 100% to the nucleotide sequence of SEQ ID NO: 3, but is not limited thereto.

The YhhS of the present application has an activity of exporting OPS, and thus the microorganism modified to enhance the YhhS activity has the property of exporting OPS, thus being useful for the production of OPS. The YhhS of the present application may be a protein having or including an amino acid sequence represented by SEQ ID NO: 4, or may be a protein consisting or consisting essentially of an amino acid sequence represented by SEQ ID NO: 4, but is not limited thereto. Additionally, the YhhS of the present application may have or include an amino acid sequence having a sequence homology or identity of 70%, 80%, 90%, 95%, or 99% or higher to the amino acid sequence represented by SEQ ID NO: 4, as long as it shows the YhhS activity. Moreover, the YhhS of the present application may consist or consist essentially of an amino acid sequence having a homology or identity of 70%, 80%, 90%, 95%, or 99% or higher to the amino acid sequence represented by SEQ ID NO: 4, but is not limited thereto. In addition, the polynucleotide encoding the YhhS may have or include a nucleotide sequence encoding the amino acid sequence represented by SEQ ID NO: 4. Further, the polynucleotide encoding the YhhS may consist or consist essentially of a nucleotide sequence encoding the amino acid sequence represented by SEQ ID NO: 4. The polynucleotide encoding YhhS of the present application may undergo various modifications in the coding region within the scope that does not change the amino acid sequence of the YhhS protein, due to codon degeneracy or in consideration of the codons preferred in an organism in which the YhhS protein is to be expressed. The polynucleotide encoding YhhS of the present application may have or include a nucleotide sequence having a homology or identity of 70%, 80%, 90%, 95%, or 99% or higher, and less than 100% to the nucleotide sequence of SEQ ID NO: 5. Additionally, the polynucleotide encoding YhhS of the present application may consist or consist essentially of a nucleotide sequence having a homology or identity of 70%, 80%, 90%, 95%, or 99% or higher, and less than 100% to the nucleotide sequence of SEQ ID NO: 5, but is not limited thereto.

In one embodiment, the microorganism including modifications to enhance the ability to produce OPS and/or the ability to export the OPS out of the cell; or to enhance the OPS-decomposing and/or influx ability may be CA07-0012 (KCCM 11212P; US 2012-0190081 A) or CA07-4821, but is not limited thereto. Regarding the contents of the OPS-producing microorganism, the disclosures in KR Patent No. 1381048 or US Publication No. 2012-0190081 may be used as references of the present application, in addition to those described above.

Another aspect of the present application provides a method for producing OPS, including culturing an OPS-producing microorganism with an enhanced NADH:quinone oxidoreductase activity in a medium.

The microorganism is as described above.

As used herein, the term "cultivation" means that the microorganism is grown under appropriately controlled environmental conditions. The cultivation process of the present application may be performed in a suitable culture medium and culture conditions known in the art. Such a cultivation process may be easily adjusted for use by those skilled in the art according to the strain to be selected. Specifically, the cultivation may be a batch culture, a continuous culture, and a fed-batch culture, but is not limited thereto.

In culturing the microorganism, the medium may further contain glycine or serine. Glycine may be provided in the form of purified glycine, a glycine-containing yeast extract, or tryptone. The concentration of glycine to be contained in the medium is generally 0.1 g/L to 10 g/L, and specifically 0.5 g/L to 3 g/L. Additionally, serine may be provided in the form of purified serine, a serine-containing yeast extract, or tryptone. The concentration of serine to be contained in the medium is generally 0.1 g/L to 5 g/L, and specifically 0.1 g/L to 1 g/L.

Examples of the carbon source to be contained in the medium may include saccharides and carbohydrates such as glucose, sucrose, lactose, fructose, maltose, starch, and cellulose; oils and fats such as soybean oil, sunflower oil, castor oil, and coconut oil; fatty acids such as palmitic acid, stearic acid, and linoleic acid; alcohols such as glycerol and ethanol; and organic acids such as acetic acid. These carbon sources may be used alone or in combination, but are not limited thereto.

Examples of the nitrogen source to be contained in the medium may include organic nitrogen sources such as peptone, yeast extract, meat gravy, malt extract, corn steep liquor, and bean flour; and inorganic nitrogen sources such as urea, ammonium sulfate, ammonium chloride, ammonium phosphate, ammonium carbonate, and ammonium nitrate. These nitrogen sources may be used alone or in combination, but are not limited thereto.

Examples of the phosphorous source to be contained in the medium may include potassium dihydrogen phosphate, dipotassium hydrogen phosphate, and corresponding sodium-containing salts, but are not limited thereto.

Additionally, the culture media may include metal salts, such as magnesium sulfate or iron sulfate, and may further contain amino acids, vitamins and appropriate precursors. These culture media or precursors may be added to the culture in the form of a batch culture or continuous culture, but are not limited thereto.

The pH of the culture may be adjusted by adding a compound such as ammonium hydroxide, potassium hydroxide, ammonia, phosphoric acid, and sulfuric acid during cultivation in an appropriate manner. Additionally, bubble formation may be prevented during the cultivation using an antifoaming agent such as fatty acid polyglycol ester. Further, oxygen gas or a gas containing oxygen may be injected to the culture in order to maintain aerobic conditions of the culture; or nitrogen gas, hydrogen gas, or carbon dioxide may be injected, or no gas may be injected, to maintain anaerobic or microaerobic conditions. The temperature of the culture may be in the range from 25°C to 40°C, specifically from 30°C to 35°C. The cultivation may be continued until the production of a useful material can be obtained, and specifically for from 10 hours to 100 hours, but is not limited to these illustrative examples.

The present application may further include a step of preparing a medium before the culturing step in the method of the present application, but is not limited thereto.

The method may further include a step of recovering OPS from the cultured medium or microorganism. The recovering step may be further included after the culturing step.

In the method of recovering OPS of the present application, the desired OPS may be collected from the culture solution using appropriate methods known in the art depending on the cultivation method. For example, methods such as centrifugation, filtration, ion exchange chromatography, crystallization, and HPLC may be used, and the desired OPS may be recovered from the medium or microorganism using a suitable method known in the art.

Further, the step of recovering may further include a purification process, which may be performed using an appropriate method known in the art. Thus, the recovered OPS may be in a purified state or in a microbial fermentation broth containing OPS. In addition, the recovery of OPS can be performed efficiently by adding a suitable method known in the art before and after the culturing step and before and after the recovery step.

Still another aspect of the present application provides a method for producing cysteine or a derivative thereof, including:
a) culturing an O-phosphoserine-producing microorganism with an enhanced NADH:quinone oxidoreductase activity in a medium to produce O-phosphoserine or a medium containing the same; and
b) reacting O-phosphoserine produced in step a) or a medium containing the same with a sulfide in the presence of O-phosphoserine sulfhydrylase (OPSS) or a microorganism expressing the same.

The steps a) and b) are not necessarily limited by the order, *i.e.,* to be performed continuously or sequentially, and there may be no time interval between these steps, and the steps may be performed simultaneously, or with an interval of several seconds, several minutes, several hours, several days.

Specifically, the method may be a method for producing cysteine or a derivative thereof: including culturing an OPS-producing microorganism with an enhanced Nuo activity in a medium to produce OPS or a medium containing the same; and reacting O-phosphoserine produced in step a) or a medium containing the same with a sulfide in the presence of O-phosphoserine sulfhydrylase (OPSS) or a microorganism expressing the same. The enhancement of the NADH:quinone oxidoreductase activity and the O-phosphoserine-producing microorganism are as described above.

As used herein, the term "derivative" refers to similar compounds obtained by chemically modifying a portion of any compound. The term usually refers to compounds in which a hydrogen atom or a particular atom group is substituted with another atom or atom group.

As used herein, the term "cysteine derivative" refers to compounds in which a hydrogen atom or a particular atom group in cysteine is substituted with another atom or atom group. For example, the cysteine derivatives may have a form in which the nitrogen atom of the amine group (-NH₂) or the sulfur atom of the thiol group (-SH) in cysteine has another atom or atom group attached thereto, and the examples of cysteine derivatives may include NAC (*N*-acetylcysteine), SCMC (S-carboxymethylcysteine), Boc-Cys(Me)-OH, (R)-S-(2-amino-2-carboxyethyl)-L-homocysteine, (R)-2-amino-3-sulfopropionic acid, D-2-amino-4-(ethylthio)butyric acid, 3-sulfino-L-alanine, Fmoc-Cys(Boc-methyl)-OH, seleno-L-cystine, S-(2-thiazolyl)-L-cysteine, S-(2-thienyl)-L-cysteine, S-(4-tolyl)-L-cysteine, *etc.,* but are not limited thereto.

As long as cysteine is produced according to the method of the present application, conversion to cysteine derivatives can be easily converted into various cysteine derivatives by a method well known in the art.

Specifically, the method of producing cysteine derivatives may further include converting the cysteine produced in step b) into a cysteine derivative. For example, cysteine may be synthesized into N-acetylcysteine (NAC) by a reaction with an acetylation agent, or it may be synthesized into S-carboxymethylcysteine (SCMC) by a reaction with a haloacetic acid in basic conditions, but is not limited thereto.

These cysteine derivatives are used mainly as pharmaceutical materials for antitussive agents, cough-relieving agents, and therapeutic agents for bronchitis, bronchial asthma, laryngopharyngitis, *etc.,* but are not limited thereto.

As used herein, the term "O-phosphoserine sulfhydrylase (OPSS)" refers to an enzyme that catalyzes a reaction by which OPS from a thiol group (-SH group) is converted into cysteine. The enzyme may have been first found in *Aeropyrum pernix, Mycobacterium tuberculosis, Mycobacterium smegmatics,* and *Trichomonas vaginalis* (Mino K. and Ishikawa K., FEBS Letters, 551:133-138, 2003; Burns K. E. et al., J. Am. Chem. Soc., 127:11602-11603, 2005). Additionally, the OPSS may include not only wild-type OPSS proteins, but also variant proteins that include deletion, substitution, or addition in part of the polynucleotide sequence encoding the OPSS which show activity that is equal to or higher than the biological activity of the wild-type OPSS proteins, and may also include all the OPSS proteins disclosed in US 2012-0190081 A1 and US 9127324 B2 and their variants.

The sulfide to be used in the present application may be any sulfide provided not only in a solid form generally used in the art, but also in a liquid or gas form due to the difference in pH, pressure, and solubility, and thus can be converted to a thiol (SH) group in the form of, for example, sulfide (S²⁻) or thiosulfate (S₂O₃²⁻) without limitation. Specifically, the sulfide may include Na₂S, NaSH, H₂S, (NH₄)₂S, NaSH, and Na₂S₂O₃, which can provide a thiol group to OPS, but is not limited thereto. In the reaction, a single thiol group is provided to a single reactive OPS group to produce a single cysteine or a derivative thereof. In this reaction, a sulfide is specifically added in an amount of 0.1 to 3 molar equivalents, and specifically 1 to 2 molar equivalents based on the molar concentration of OPS, but is not limited thereto.

Yet another aspect of the present application provides a method for producing an OPS-producing microorganism, including: modifying a microorganism of the genus *Escherichia* to enhance the activity of NADH:quinone oxidoreductase.

Even another object of the present application provides the use for the production of O-phosphoserine, cysteine, or a derivative thereof of a microorganism having an enhanced NADH:quinone oxidoreductase activity.

The NADH:quinone oxidoreductase, its activity enhancement, microorganisms, *etc.* are the same as described above.

### [Mode for Carrying Out the Invention]

The present application will be described in more detail by way of Examples. However, it is apparent to those skilled in the art to which the present application belongs that these Examples are provided for illustrative purposes only, and that the scope of the invention is not intended to be limited to or by these Examples.

### Example 1: Evaluation of OPS Producing Ability of Strain with Mutated Nuo Expression Regulatory Region

As a result of the previous study, it was found that the average transcription levels of the nuoA, rmf, and idi genes in the OPS-producing host strain were 8986, 32205, and 631, respectively, and the specific values for each culture section are shown in Table 1.

**[Table 1]**

| Gene name | Early Exponential | Mid Exponential | Late Exponential | Stationary | Average |
|---|---|---|---|---|---|
| *idi* | 914 | 740 | 637 | 234 | 631 |
| *nuoA* | 6969 | 7517 | 8535 | 12922 | 8986 |
| *rmf* | 30725 | 28192 | 28109 | 41792 | 32205 |

It was confirmed that the average value of rmf transcription level was 3.6 times that of nuoA, and the average value of idi transcription level was 0.07 times that of nuoA. These results indicate that the promoter of the rmf gene is a strong promoter, and the promoter of the idi gene is a weak promoter, compared to the promoter of nuoA.

Therefore, it was attempted to compare the OPS producing ability of microorganisms when the expression of NADH:quinone oxidoreductase was regulated by inserting the rmf promoter and the idi promoter with different activities into the nuo operon (SEQ ID NO: 1) within the OPS-producing microorganism.

### 1-1: Construction of Plasmid for Enhancing Nuo Expression Regulatory Region

The gene fragments in the upstream region of the nuo gene wild-type promoter were obtained using a primer pair having the nucleotide sequences of SEQ ID NOS: 14 and 15, and the gene fragments in the downstream region of the promoter were obtained using a primer pair having the nucleotide sequences of SEQ ID NOS: 18 and 19 based on the *E. coli* ATCC27325 chromosomal DNA as a template. In addition, the rmf gene promoter region was obtained using a primer pair having the nucleotide sequences of SEQ ID NOS: 16 and 17 based on the E. *coli* ATCC27325 chromosomal DNA as a template.

In order to obtain the fragments, PCR was performed using Solg^{™} Pfu-X DNA polymerase, and the PCR was performed under PCR amplification conditions of denaturation at 95°C for 2 minutes, followed by 30 cycles of denaturation at 95°C for 30 seconds, annealing at 60°C for 30 seconds, and polymerization at 72°C for 60 seconds, and then polymerization at 72°C for 5 minutes.

The upstream fragments and downstream fragments of the nuo promoter, and the rmf promoter fragments obtained by the above process were cloned with the pSKH130 vector (SEQ ID NO: 39, U.S. Publication No. 2020-0048619) for chromosomal transformation cleaved with the restriction enzyme EcoRV, using an in-fusion cloning kit (Clontech Laboratories, Inc.) to obtain a recombinant plasmid, and it was named pSKH130_Prmf-nuoA.

### 1-2: Construction of Plasmid for Weakening Nuo Expression Regulatory Region

The gene fragments in the upstream region of the nuo gene wild-type promoter were obtained using a primer pair having the nucleotide sequences of SEQ ID NOS: 14 and 20, and the gene fragments in the downstream region of the promoter were obtained using a primer pair having the nucleotide sequences of SEQ ID NOS: 23 and 19 based on the *E. coli* ATCC27325 chromosomal DNA as a template. In addition, the idi gene promoter region was obtained using a primer pair having the nucleotide sequences of SEQ ID NOS: 21 and 22 based on the *E. coli* ATCC27325 chromosomal DNA as a template.

PCR was performed under the same PCR conditions as in Example 1-1 to obtain the fragments, and cloning was performed in the same manner as in Example 1-1 using the fragments to obtain a recombinant plasmid. This was named pSKH130_Pidi-nuoA.

The primer sequences used in Examples 1-1 and 1-2 are shown in Table 2.

**[Table 2]**

| SEQ ID NO: | Name | Sequence (5'→3') |
|---|---|---|
| 14 | UP F1 | CTGCAGGAATTCGATCAACCACCAGAGATTCACGT |
| 15 | UP R1 | GCTTCCTGACTCCAGTGCTTACTCATCAAAAGTAG |
| 16 | Prmf F | TTTGATGAGTAAGCACTGGAGTCAGGAAGCCGCTT |
| 17 | Prmf R | TGTTGACATACTCATGCCTCGTTTCCCTCATACTG |
| 18 | Down F1 | TGAGGGAAACGAGGCATGAGTATGTCAACATCCAC |
| 19 | Down R1 | ACGGTGAGAACGACCGTTACATCACGCTAGTCGAC |
| 20 | UP R2 | TCATTTTTCTTCAGCTGCTTACTCATCAAAAGTAG |
| 21 | Pidi F | TTTGATGAGTAAGCAGCTGAAGAAAAATGAGCATG |
| 22 | Pidi R | TGTTGACATACTCATAATTTCTCACATGTAATTCTGA TC |
| 23 | Down F2 | TACATGTGAGAAATTATGAGTATGTCAACATCCAC |

### 1-3: Construction of Nuo Expression Regulatory Region Mutant Strain

The pSKH130_Prmf-nuoA prepared in Example 1-1 was transformed into the CA07-0012 (KCCM 11212P, U.S. Publication No. 2012-0190081) having OPS producing ability by electroporation *(*Appl. Microbiol. Biotechnol. (1999) 52:541-545) and subjected to secondary crossover to thereby obtain CA07-4826 strain into which the promoter nucleotide sequence of the rmf gene was inserted at the terminal of the wild-type promoter nucleotide sequence of the nuo gene.

Specifically, the pSKH130 vector contained a PI protein (pir gene)-dependent R6K replicon, SacB (Levansucrase) gene, and kanamycin resistance gene. After obtaining the desired strain using R6K and kanamycin at the first crossover, the antibiotics were removed from the medium containing sucrose to prepare a strain.

In the CA07-4826, the insertion of the rmf promoter nucleotide sequence was confirmed through PCR and genome sequencing using the primer pair of SEQ ID NOS: 24 and 25.

In the same manner, the pSKH130_Pidi-nuoA prepared in Example 1-2 was transformed by electroporation *(*Appl. Microbiol. Biotechnol. (1999) 52:541-545) and subjected to secondary crossover to thereby obtain CA07-4827 strain into which the promoter nucleotide sequence of the idi gene was inserted at the terminal of the wild-type promoter nucleotide sequence of the nuo gene. In the CA07-4827, the insertion of the idi promoter nucleotide sequence was confirmed through PCR and genome sequencing using the primer pair of SEQ ID NOS: 24 and 25. The primer sequences used are shown in Table 3.

**[Table 3]**

| SEQ ID NO: | Name | Sequence (5'→3') |
|---|---|---|
| 24 | NuoP Conf F | GAGAGCTACCATAATCCGTG |
| 25 | NuoP Conf R | CATCTCAACGTAACAGCAGG |

### 1-4: Comparison of OPS Producing Ability of nuo Operon-Enhanced and Weakened Strains Using Titer Medium

In order to evaluate the OPS producing ability of the two strains CA07-4826 and CA07-4827 prepared in Example 1-3, and the control CA07-0012, the following medium (Table 4) was used for evaluation.

**[Table 4]**

| Medium Component | Amount |
|---|---|
| Glucose | 40 g |
| KH₂PO₄ (KP1) | 6 g |
| (NH₄)₂SO₄ | 17g |
| MgSO₄·7H₂O | 1 g |
| MnSO₄·4H₂O | 5 mg |
| FeSO₄·7H₂O | 10 mg |
| L-Glycine | 1.5 g/L |
| Yeast Extract | 2.5 g/L |
| CaCO₃ | 30 g/L |
| pH | 6.8 |

Specifically, each of the strains was plated out on a solid LB medium and cultured in a 33°C incubator overnight. The strains cultured in the solid LB medium overnight were inoculated into a 25 mL titer medium shown in Table 3 below and then cultured in an incubator at a rate of 200 rpm for 48 hours at 33°C. The concentration of OPS produced is shown in Table 5.

**[Table 5]**

| Name of Strains | OPS (g/L) |
|---|---|
| CA07-0012 | 1.95 |
| CA07-4826 | 2.20 |
| CA07-4827 | 0.40 |

The CA07-4826, in which the nuo operon was enhanced by the rmf promoter, showed an improved OPS producing ability by about 12.8% compared to the parent strain, and the CA07-4827, in which the nuo operon was weakened by the idi promoter, showed a reduced OPS producing ability by about 79.5% compared to the parent strain.

### Example 2: Evaluation of OPS Producing Ability According to Enhancement of nuo Operon in Strain with Enhanced OPS-Producing Ability

It was attempted to confirm whether the OPS producing ability was further increased when the Nuo operon was enhanced in the strain with an enhanced OPS exporting ability. To this end, the OPS producing ability was evaluated when the nuo operon was further enhanced in the strain enhanced with the protein YhhS (SEQ ID NO: 4, U.S. Patent No. 10323262 B2) having an OPS exporting ability.

### 2-1: Construction of Plasmid for Enhancement of YhhS in OPS-Producing Strain

The gene fragments in the upstream region of the yhhS gene wild-type promoter were obtained using a primer pair having the nucleotide sequences of SEQ ID NOS: 6 and 7, and the gene fragments in the downstream region of the yhhS gene wild-type promoter were obtained using a primer pair having the nucleotide sequence of SEQ ID NOS: 8 and 9 based on the *E. coli* ATCC27325 chromosomal DNA as a template. In addition, the trc promoter (Ptrc) was obtained using a primer pair of SEQ ID NOS: 8 and 9 based on the pCL_Ptrc-gfp(US 2017-0247727 A1) as a template. The primer sequences used are shown in Table 6 below.

**[Table 6]**

| SEQ ID NO: | Name | Sequence (5'→3') |
|---|---|---|
| 6 | yhhS UP F | CTGCAGGAATTCGATGAAGGTAACCACTTTTTCCG |
| 7 | yhhS UP R | GAGTTGCAGCAAGCGGAGGATCACCACATTTTTAC |
| 8 | Ptrc F | AATGTGGTGATCCTCCGCTTGCTGCAACTCTCTCA |
| 9 | Ptrc R | TACGGGTTCGGGCATGATAGCTCTCCTGTGTGAAA |
| 10 | yhhS Down F | CACAGGAGAGCTATCATGCCCGAACCCGTAGCCG A |
| 11 | yhhS Down R | GTCGACTAGCGTGATAGCGGTTTGCCTTTACTGGC |

In order to obtain the fragments, PCR was performed using Solg^{™} Pfu-X DNA polymerase, and the PCR was performed under PCR amplification conditions of denaturation at 95°C for 2 minutes, followed by 30 cycles of denaturation at 95°C for 30 seconds, annealing at 60°C for 30 seconds, and polymerization at 72°C for 60 seconds, and then polymerization at 72°C for 5 minutes.

The upstream fragments and downstream fragments of the yhhS promoter, and the trc promoter fragment obtained by the above process, were cloned with the vector for chromosomal transformation cleaved with the restriction enzyme EcoRV, using an in-fusion cloning kit (Clontech Laboratories, Inc.) to obtain a recombinant plasmid, and it was named pSKH130_Ptrc-yhhS.

### 2-2: Construction of YhhS-Enhanced Strain

The pSKH130_Ptrc-yhhS prepared in Example 2-1 was transformed into the CA07-0012 by electroporation *(*Appl. Microbiol. Biotechnol. (1999) 52:541-545) and subjected to secondary crossover to thereby obtain CA07-4821 strain into which the trc promoter nucleotide sequence was inserted at the terminal of the wild-type promoter nucleotide sequence of the yhhS gene. In the CA07-4821 strain, the insertion of the trc promoter nucleotide sequence was confirmed through PCR and genome sequencing using the primer pair of SEQ ID NOS: 12 and 13 (Table 7).

**[Table 7]**

| SEQ ID NO: | Name | Sequence (5'→3') |
|---|---|---|
| 12 | yhhSP Conf F | AGTTGAGCAAAAACGCCAGT |
| 13 | yhhSP Conf R | GCCATACCGTACAGCCAGAC |

### 2-3: Evaluation of OPS Producing Ability of YhhS-Enhanced Strain Using Titer Medium

In order to evaluate the OPS producing ability of the strain CA07-4821 prepared in Example 2-2 and the parent strain CA07-0012 thereof, the evaluation was carried out in the same manner as in Example 1-4 using the medium (Table 4).

As a result, it was confirmed that CA07-4821 showed an improved OPS producing ability by about 26.7% compared to CA07-0012, and the results are shown in Table 8.

**[Table 8]**

| Name of Strain | OPS (g/L) |
|---|---|
| CA07-0012 | 1.95 |
| CA07-4821 | 2.47 |

### 2-4: Construction of Strain with Enhanced YhhS and nuo Operon

The pSKH130_Prmf-nuoA prepared in Example 1-1 was transformed into the CA07-4821 prepared in Example 2-3 by electroporation *(*Appl. Microbiol. Biotechnol. (1999) 52:541-545) and subjected to secondary crossover to thereby obtain CA07-4828 strain into which the promoter nucleotide sequence of the rmf gene was inserted at the terminal of the wild-type promoter nucleotide sequence of the nuo gene.

In the CA07-4828, the insertion of the rmf promoter nucleotide sequence was confirmed through PCR and genome sequencing using the primer pair of SEQ ID NOS: 24 and 25.

### 2-5: Evaluation of OPS Producing Ability of Strain with Enhanced Yhhs and nuo Operon Using Titer Medium

In order to evaluate the OPS producing ability of CA07-4828, the evaluation was carried out in the same manner as in Example 1-4 using CA07-4821 as a control, and the results are shown in Table 9.

**[Table 9]**

| Name of Strain | OPS (g/L) |
|---|---|
| CA07-4821 | 2.47 |
| CA07-4828 | 2.53 |

In the case of CA07-4828, in which YhhS and nuo operon were simultaneously enhanced, the OPS producing ability was improved by about 2.4% compared to CA07-4821, in which only YhhS was enhanced. Thus, it was confirmed that the enhancement of the nuo operon further increased the OPS producing ability even in the strain with an enhanced OPS producing ability.

From the foregoing, a skilled person in the art to which the present application pertains will be able to understand that the present application may be embodied in other specific forms without modifying the technical concepts or essential characteristics of the present application. In this regard, the exemplary embodiments disclosed herein are only for illustrative purposes and should not be construed as limiting the scope of the present application. On the contrary, the present application is intended to cover not only the exemplary embodiments but also various alternatives, modifications, equivalents, and other embodiments that may be included within the spirit and scope of the present application as defined by the appended claims.

## Claims

1. A recombinant microorganism of the genus *Escherichia* having an enhanced NADH:quinone oxidoreductase activity and an O-phosphoserine producing ability.

2. The microorganism of claim 1, wherein the enhancement of the NADH:quinone oxidoreductase activity is achieved by an increase in the expression of nuo operon.

3. The microorganism of claim 1, wherein the enhancement of the NADH:quinone oxidoreductase activity comprises a gene expression regulatory sequence with enhanced activity in the upstream of the gene encoding the NADH:quinone oxidoreductase.

4. The microorganism of claim 3, wherein the upstream of the gene encoding the NADH:quinone oxidoreductase is the upstream of nuoA gene.

5. The microorganism of claim 1, wherein the activity of phosphoserine phosphatase (SerB) is further weakened.

6. The microorganism of claim 1, wherein the activity of O-phosphoserine export protein (YhhS) is further enhanced.

7. The microorganism of claim 1, wherein the microorganism is *Escherichia coli.*

8. A method for producing *O*-phosphoserine, comprising: culturing the microorganism of any one of claims 1 to 7 in a medium.

9. The method of claim 8, wherein the method further comprises recovering *O-*phosphoserine from the cultured medium or microorganism.

10. A method for producing cysteine or a derivative thereof, comprising:
a) culturing an *O*-phosphoserine-producing microorganism with an enhanced NADH:quinone oxidoreductase activity in a medium to produce *O*-phosphoserine or a medium containing the same; and
b) reacting *O*-phosphoserine produced in step a) or a medium containing the same with a sulfide in the presence of *O*-phosphoserine sulfhydrylase (OPSS) or a microorganism expressing the same.

11. The method of claim 10, wherein the sulfide is at least one selected from the group consisting of Na₂S, NaSH, (NH₄)₂S, H₂S, and Na₂S₂O₃.
